# EUROPEAN PATENT APPLICATION

(11) **EP 4 169 606 A1**
(43) Date of publication of application: **26.04.2023**
(21) Application number: 21827050.2
(22) Date of filing: 15.06.2021
(51) Int. Cl.: B01F 3/02, B01F 5/00, C01B 13/10, B01D 53/48, B01D 53/52, B01D 53/54, B01D 53/58, B01D 53/72, B01D 53/76

(54) **GAS TREATMENT METHOD AND GAS TREATMENT DEVICE**

(30) Priority: 17.06.2020 JP 2020104649
(71) Applicant: Ushio Denki Kabushiki Kaisha, Tokyo 100-8150 (JP)
(72) Inventor: OTSUKA,Yuichi, Tokyo 100-8150 (JP); HIRAOKA,Takahiro, Tokyo 100-8150 (JP); MIURA,Masaki, Tokyo 100-8150 (JP); NAKAMURA,Kensuke, Tokyo 100-8150 (JP)
(74) Representative: Tomerius, Isabel
(86) International application number: PCT/JP2021/022608
(87) International publication number: WO 2021/256450

(57) **Abstract**

A gas treatment method includes: a process (a) of allowing gas to be treated in which a target substance to be treated is mixed with air to pass through inside a housing, the target substance to be treated exhibiting volatility at room temperature and belonging to at least one substance selected from a group consisting of carbon compounds, nitrogen compounds, and sulfur compounds; a process (b) of introducing ozone into a space through which the gas to be treated flows inside the housing at 200°C or lower; a process (c) of stirring the gas to be treated after the process (b); and a process (d) of heating the gas to be treated to 300°C or higher after executing the process (c).

## Description

### TECHNICAL FIELD

The present invention relates to a gas treatment method and a gas treatment device, and in particular, to a method and device for treating gas to be treated in which a target substance to be treated exhibiting volatility at room temperature is mixed with air.

### BACKGROUND ART

With global warming in progress, a greenhouse gas that contributes to global warming is known to include carbon dioxide, methane, and chlorofluorocarbon gas. The amount of carbon dioxide emissions is the largest and the amount of methane emissions is the second largest. The global warming potential of methane is considered to be about 20 to 70 times that of carbon dioxide, thus its impact on global warming is significant. In addition, the concentration of methane has been increasing in recent years, hence technology of efficiently decomposing methane that has been emitted will be necessary in view of the future of the global environment.

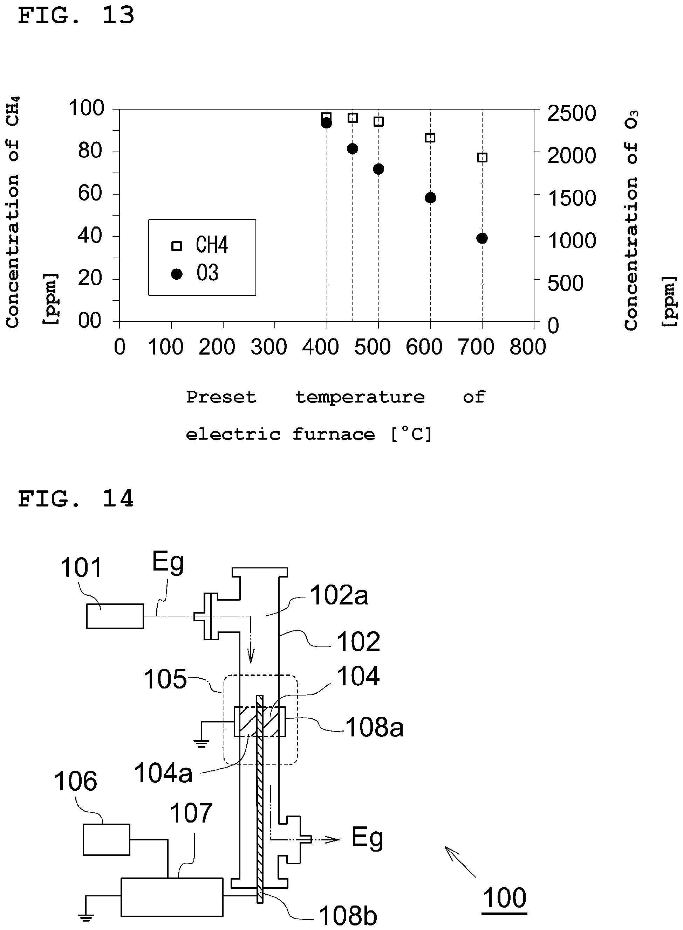

Patent Document 1 discloses a methane removal system as a conventional technology of decomposing methane. FIG. 14 is a drawing schematically illustrating a configuration of the methane removal system.

A methane removal system 100 is a system that decomposes methane contained in gas to be treated, and includes a tube for gas to be treated 102, a catalyst for removing methane by oxidation 104, a plasma generation means 105, and a control means 106.

A tube for gas to be treated 102 is configured inside a channel for gas to be treated 102a through which gas to be treated Eg is exhausted from an emission source of gas to be treated 101. A catalyst for removing methane by oxidation 104 that has in layers is accommodated in the catalyst container 104a that is configured inside the channel for gas to be treated 102a, and is used to remove the gas to be treated Eg that flows inside the channel for gas to be treated 102a.

The plasma generation means 105 includes an external electrode 108a, an internal electrode 108b, and a power supply source 107. The external electrode 108a is disposed around the outer circumference of the tube for gas to be treated 102 in a manner that surrounds the catalyst container 104a, and has a cylindrical shape. The internal electrode 108b is disposed to be located at a position corresponding to the catalyst container 104a in the channel for gas to be treated 102a. The power supply source 107 has one terminal electrically connected to the internal electrode 108b. The other terminal of the power supply source 107 and the external electrode 108a are grounded.

The plasma generation means 105 supplies power to the internal electrodes 108b by the power supply source 107 to generate atmospheric pressure plasma at a position where the catalyst for removing methane by oxidation 104 accommodated in the catalyst container 104a exists. This allows the gas to be treated Eg circulating in the channel for gas to be treated 102a to transform into plasma in the catalyst container 104a. The generated plasma activates the catalyst for removing methane by oxidation 104. The methane contained in the gas to be treated Eg is decomposed into carbon dioxide by the synergy of the action of the catalyst for removing methane by oxidation 104 that has been activated and the action of the generated plasma.

### CITATION LIST

### PATENT DOCUMENTS

Patent Document 1: JP-A-2019-155242

### SUMMARY OF INVENTION

### Technical Problem

The largest source of methane emissions released into the environment is reportedly livestock and other animals in the form of farts and burps. For example, the amount of methane emissions released from a single cow is estimated to be about 300 liters per day. On the other hand, the atmospheric concentration of methane released from cattle and other livestock raised in cattle sheds is about 20 ppm.

The Patent Document 1 discloses that methane was able to be decomposed by allowing gas to be treated having a methane concentration of 3000ppm to pass through the system. However, the technology that can decompose methane having low concentrations such as several hundred ppm or less, has yet to been sufficiently established at the present moment.

In the case of decomposing methane from gas to be treated containing methane at high concentrations (e.g., several thousand ppm or more, or several percent or less), a method of simply combusting gas to be treated is considered to be the simplest and most effective method. However, in the case of such a combustion process, the residual gas often contains methane at several hundred ppm or less. Decomposing methane by combustion process for the treated gas containing methane at such low concentrations is impractical method from the viewpoint of the decomposition amount of methane with respect to the amount of energy input.

Incidentally, in addition to the viewpoint of global warming, there exists a demand of treating air containing substances that cause odors. For example, the above-mentioned cattle sheds emit gas with odors such as ammonia, hydrogen sulfide, methyl mercaptan, etc., in addition to methane. Hence, the atmospheric gas from cattle sheds comes to contain these substances in low concentrations. Moreover, for gas exhausted from locations other than cattle sheds, there exists a similar demand of treating air containing odor causing substances at low concentrations.

In view of the above issues, it is an object of the present invention that provides a technology that can decompose, by a simple method, gas to be treated containing target substance to be treated at low concentrations such as several hundred ppm or less.

### Solution to the problem

A gas treatment method according to the present invention includes:
a process (a) of allowing gas to be treated in which a target substance to be treated is mixed with air to pass through inside a housing, the target substance to be treated exhibiting volatility at room temperature and belonging to at least one substance selected from a group consisting of carbon compounds, nitrogen compounds, and sulfur compounds;
a process (b) of introducing ozone into a space through which the gas to be treated flows inside the housing at 200°C or lower;
a process (c) of stirring the gas to be treated after the process (b); and
a process (d) of heating the gas to be treated to 300°C or higher after executing the process (c).

The details will be described later; however, the diligent study of the present inventors confirmed that the decomposition rate of the target substance to be treated contained in the gas to be treated at low concentrations such as several hundred ppm or less is improved by introducing ozone into the gas to be treated at 200°C or less, stirring the gas to be treated, and then performing heat treatment.

Stirring the gas to be treated after the introduction of ozone increases the probability of contact of the gas to be treated with ozone. Subsequently conducting heat treatment makes O(³P) that is produced by the thermal decomposition of ozone easier to come into contact with the gas to be treated, increasing the decomposition rate of the target substance to be treated contained in the gas to be treated.

The target substance to be treated may belong to at least one substance selected from a group consisting of methane, acetylene, ethylene, ethane, propane, methylamine, ammonia, hydrogen sulfide, and methyl mercaptan.

The process (c) may be a process of allowing the gas to be treated to pass through a stirring area in which a cross-sectional area of a gas channel is varied, or a stirring area in which a wind shield plate is provided in the middle of the gas channel.

This enables the gas to be treated to be stirred while decreasing the velocity of the gas to be treated flowing toward the outlet through which the gas that has been treated is exhausted after ozone has been introduced.

The stirring area may have a length longer than the length of the area through which the gas to be treated flows when the process (b) is executed, in a direction of flow of the gas to be treated.

The above method makes the gas to be treated easier to come into contact with ozone before executing the heating process (c). As a result, O(³P) that is produced by the thermal decomposition of ozone in the heating process (c), which is to be executed later, is made easier to come into contact with the gas to be treated.

A gas treatment device according to the present invention is a gas treatment device that treats gas to be treated in which a target substance to be treated is mixed with air, the target substance to be treated exhibiting volatility at room temperature and belonging to at least one substance selected from a group consisting of carbon compounds, nitrogen compounds, and sulfur compounds, the gas treatment device includes:
a housing;
a gas inlet through which the gas to be treated is introduced into the housing;
an ozone introduction unit that introduces ozone into a gas channel through which the gas to be treated flows inside the housing;
a stirring area that is disposed downstream from a location of the ozone introduction unit, and that stirs the gas to be treated flowing through the gas channel;
a heating area that is disposed downstream from a location of the stirring area, and that heats the gas to be treated flowing through the gas channel; and
a gas outlet through which gas that has been treated having passed through the heating area is exhausted from the housing.

In the above gas treatment device, the gas to be treated and ozone that is introduced by the ozone introduction unit come into contact with each other at a high probability when passing through the stirring area, and then are introduced into the heating area. As a result, O(³P) produced by the thermal decomposition of ozone comes into contact with the gas to be treated at a high probability, allowing the target substance to be treated contained in the gas to be treated to decompose. Then, the gas that has been treated in which the target substance to be treated is decomposed with high efficiency is exhausted from the gas outlet.

The gas channel in the stirring area in the direction of flow of the gas to be treated may have a length longer than a length from a location at which ozone is introduced by the ozone introduction unit to the stirring area in the direction of flow of the gas to be treated.

The above configuration enables the gas to be treated passing through the stirring area to come into contact with ozone at higher probability.

The gas channel may be configured to include a heated wall whose surface is heated to 300°C or higher in the heating area.

An example of a more specific configuration includes an aspect in which the entire heating area is mounted in the heating furnace. Another example includes an aspect in which a heat transfer member such as a metal tube is disposed on the inner wall surface of the gas channel located in the heating area, and this heat transfer member is heated. Yet another example includes an aspect in which a heated plate member having an opening is mounted in the gas channel, and the gas to be treated flows through the opening.

The gas channel may be bent in the heating area.

In the above configuration, when the gas to be treated flows through the heating area, its flow velocity is reduced at a bent section, thereby extending the time for the gas to be treated to flow through the heating area. This allows the heating time of the gas to be treated containing ozone to be longer, thereby increasing the time for O(³P), which is obtained by the thermal decomposition of ozone, to come into contact with the gas to be treated, in other words, the reaction time between the target substance to be treated contained in the gas to be treated and O(³P). This further increases the decomposition rate of the target substance to be treated.

The gas channel may be configured in a manner that a cross-sectional area of the gas channel changes in the stirring area.

In the above configuration, when the gas to be treated flows through the location in which the cross-sectional area of the gas channel changes, turbulence is readily generated due to the pressure difference. This turbulence causes the gas to be treated and ozone to be sufficiently stirred and mixed, making the ozone and the gas to be treated readily come into contact with each other.

The gas channel may include a wind shield plate with which the gas to be treated flowing through the gas channel collides in the stirring area.

In the above configuration, when the gas to be treated collides with the wind shield plate, the direction of airflow changes, readily generating turbulence at the position. This turbulence causes the gas to be treated and ozone to be sufficiently stirred and mixed, making the ozone and the gas to be treated readily come into contact with each other.

The ozone introduction unit may include a light source that is disposed in the gas channel, and that emits ultraviolet light having a main peak wavelength of less than 200 nm, and ozone may be generated from part of the gas to be treated by irradiating the gas to be treated with the ultraviolet light from the light source.

In the above configuration, ozone can be generated from gas to be treated without separately supplying gas as an ozone generation source. In particular, when the light source is configured to be an excimer lamp using Xe as its luminescent gas, the main peak wavelength is near 172 nm (160 nm or more and less than 180 nm), thereby also providing a secondary effect that no NOₓ is generated during ozone generation.

The ozone introduction unit may include an atmospheric pressure plasma generator that is disposed in the gas channel, and ozone may be generated from part of the gas to be treated by allowing the gas to be treated to pass through a space of atmospheric pressure plasma generated by the atmospheric pressure plasma generator.

In the above configuration, ozone can be generated from gas to be treated without separately supplying gas as an ozone generation source.

The ozone introduction unit may include an ozone generator that is disposed in a channel different from the gas channel, and ozone gas generated from the ozone generator may be supplied into the gas channel.

### Advantageous Effects of Invention

According to the present invention, gas to be treated containing a target substance to be treated at low concentrations such as several hundred ppm or less, can be decomposed by a simple method.

### BRIEF DESCRIPTION OF DRAWINGS

FIG. 1 is a cross-sectional view schematically illustrating a configuration of a first embodiment of a gas treatment device.
FIG. 2A is a side view schematically illustrating an example of the configuration of an excimer lamp as an ozone introduction unit.
FIG. 2B is a cross-sectional view taken along the line A1-A1 in FIG. 2A.
FIG. 3 is a graph indicating the spectrum of ultraviolet light emitted from an excimer lamp filled with luminescent gas containing Xe and the absorption spectrum of oxygen (O2) in a superimposed manner.
FIG. 4A is a drawing schematically illustrating an example of the configuration of a stirring area.
FIG. 4B is a drawing schematically illustrating another example of the configuration of a stirring area.
FIG. 4C is a drawing schematically illustrating yet another example of the configuration of a stirring area.
FIG. 5 is a cross-sectional view schematically illustrating another configuration of the first embodiment of the gas treatment device.
FIG. 6 is a graph illustrating a relationship between the thermal decomposition rate (half-life) of ozone and temperature.
FIG. 7 is a graph illustrating a relationship between the reaction rate in formulas (7) and (9), and temperature.
FIG. 8A is a drawing schematically illustrating another example of the configuration of a heating area.
FIG. 8B is a drawing schematically illustrating yet another example of the configuration of a heating area.
FIG. 9A is a cross-sectional view schematically illustrating another example of the configuration of an excimer lamp as an ozone introduction unit.
FIG. 9B is a plan view schematically illustrating another example of the configuration of an excimer lamp as an ozone introduction unit.
FIG. 9C is a cross-sectional view taken along the line A2-A2 in FIG. 9B.
FIG. 10A is a cross-sectional view schematically illustrating a configuration of a second embodiment of the gas treatment device.
FIG. 10B is another cross-sectional view schematically illustrating the configuration of the second embodiment of the gas treatment device.
FIG. 11A is a cross-sectional view schematically illustrating a configuration of an atmospheric pressure plasma generator as an ozone introduction unit.
FIG. 11B is a cross-sectional view taken along the line A3-A3 in FIG. 11A.
FIG. 12 is a drawing schematically illustrating a structure of an experimental system used in Examples.
FIG. 13 is a graph illustrating a relationship between the concentrations of methane and ozone in the gas that has been treated in Examples 1 through 5 and the preset temperatures of an electric furnace.
FIG. 14 is a drawing schematically illustrating a configuration of a conventional methane removal system.

### DESCRIPTION OF EMBODIMENTS

Embodiments of a gas treatment method and a gas treatment device according to the present invention will be described with reference to the drawings. Note that the following drawings are schematically illustrated, and a dimensional ratio on the drawing does not always match an actual dimensional ratio. Furthermore, the dimensional ratios between the drawings do not always the same.

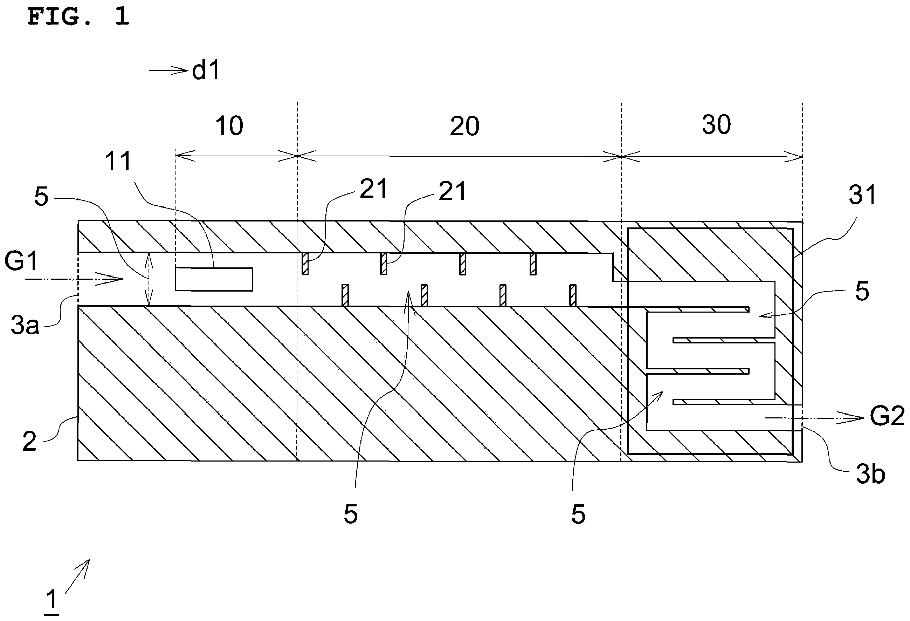

FIG. 1 is a cross-sectional view schematically illustrating a configuration of a first embodiment of a gas treatment device. A gas treatment device 1 is a device that treats gas to be treated G1 in which a target substance to be treated is mixed with air, in the device and exhausts it as gas that has been treated G2. In FIG. 1, the direction of flow of the gas to be treated G1 is indicated as d1.

The target substance to be treated belongs to at least one substance selected from a group consisting of carbon compounds, nitrogen compounds, and sulfur compounds. Examples of the target substance to be treated include methane, acetylene, ethylene, ethane, propane, methylamine, ammonia, hydrogen sulfide, methyl mercaptan, which exhibit volatility at room temperature and exhibit an odor.

The gas treatment device 1 includes a housing 2, a gas inlet 3a through which the gas to be treated G1 is introduced into the housing 2, and a gas outlet 3b through which gas that has been treated G2, which is gas after the gas to be treated G1 has been treated in the housing 2, is exhausted from the housing 2. The housing 2 is provided with a gas channel 5 through which the gas to be treated G1 (or the gas that has been treated G2) is allowed to flow.

The gas to be treated G1 that is introduced into the housing 2 from the gas inlet 3a is expected to be at room temperature or below approximately 100 °C; the temperature thereof will not exceed 200 °C no matter how high it is. In contrast, the gas to be treated G1 that has been introduced into the heating area 30, which will be described below, is heated to 300°C or higher.

In the present embodiment, the gas treatment device 1 houses an excimer lamp 11. This excimer lamp 11 constitutes an ozone introduction unit that introduces ozone into the gas channel 5. In the following description, the area where ozone is introduced into the gas channel 5 from the ozone introduction unit is referred to as the "ozone introduction area 10".

### Ozone introduction unit

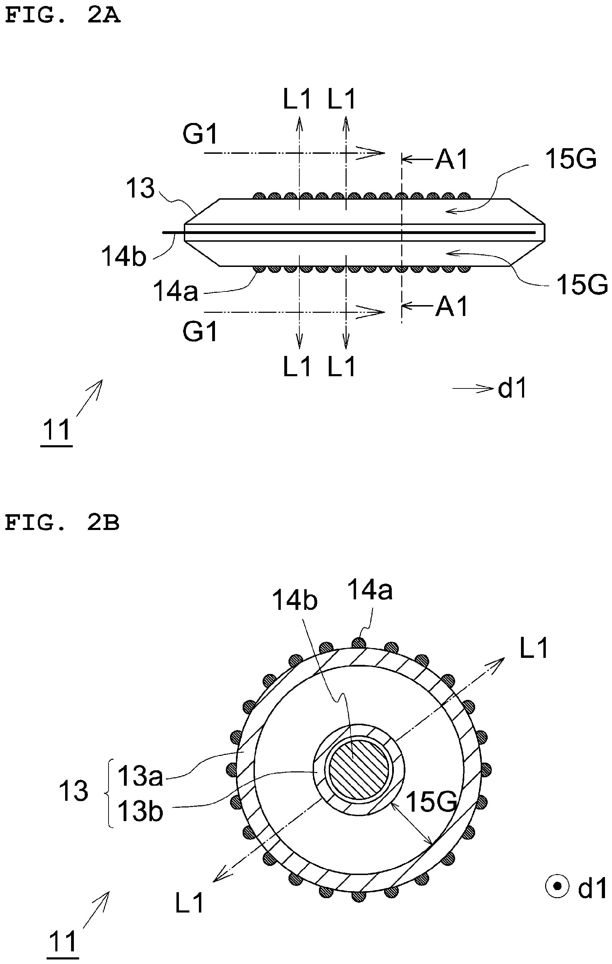

FIGS. 2A and 2B are drawings schematically illustrating an example of the configuration of an excimer lamp 11. FIG. 2A corresponds to a side view of the excimer lamp 11, and FIG. 2B corresponds to a cross-sectional view taken along the line A1-A1 in FIG. 2A.

The excimer lamp 11 illustrated in FIGS. 2A and 2B is provided with a tube body 13 and a pair of electrodes (14a, 14b). In the present embodiment, the tube body 13 has a double-tube structure. More specifically, as shown in FIG. 2B, the tube body 13 includes an outer tube 13a and an inner tube 13b. The outer tube 13a is located on the outside and has a cylindrical shape. The inner tube 13b is located coaxially with the outer tube 13a inside the outer tube 13a, and has a cylindrical shape. The inner diameter of the outer tube 13a is larger than the outer diameter of the inner tube 13b.

In the excimer lamp 11 shown in FIGS. 2A and 2B, presented is an example in which the tube body 13 is disposed such that a direction of its tube axis is aligned with a direction of flow d1 of the gas to be treated G1. However, the direction of the tube axis of the tube body 13 is not necessarily parallel to the direction of flow d1 of gas to be treated; this drawing is merely an example. Hereinafter, for convenience, the direction of flow d1 of the gas to be treated G1 is assumed to be parallel to the direction of the tube axis of tube body 13. The sign "d1" is also used for the direction of the tube axis, which is same as the direction of flow, in the viewpoint of avoiding an increase in the number of signs.

The outer tube 13a and the inner tube 13b are both sealed at their ends in the direction of the tube axis d1 (not shown). A luminescent space with a circular shape (in this case, an annular shape) is formed between them when viewed from the direction of the tube axis d1. This luminescent space is filled with luminescent gas 15G that forms excimer molecules by electrical discharge.

An electrode 14a is provided on the outer wall of the outer tube 13A. In the present embodiment, this electrode 14a has a mesh shape or a linear shape. Also, a rod-shaped electrode 14b extending along the direction of the tube axis of the tube body 13 is inserted inside the inner tube 13b.

The outer tube 13a and inner tube 13b exhibit transparency to ultraviolet light L1, and are made of a dielectric material such as synthetic quartz glass, for example. The electrodes (14a, 14b) are made of a metal material including stainless steel, aluminum, copper, tungsten, titanium, nickel.

When a high-frequency AC voltage of approximately 1 kHz to 5 MHz is applied between the electrodes (14a, 14b) via a power feed line from a power supply (not shown), the voltage is applied to the luminescent gas 15G via the tube body 13. This generates a discharge plasma in the discharge space in which the luminescent gas 15G is sealed, which causes the atoms of the luminescent gas 15G to be excited to the excimer state, producing excimer luminescence during the transition of these atoms to the ground state.

The material of the luminescent gas 15G determines the wavelength of the ultraviolet light L1 emitted from the tube body 13. When gas containing xenon (Xe) is used as the luminescent gas 15G, the excimer luminescence is the ultraviolet light L1 having a main peak wavelength near 172nm.

The wavelength of the ultraviolet light L1 can be changed by using different substances as the luminescent gas 15G. Examples of the luminescent gas 15G include ArBr (main peak wavelength being near 165 nm), ArCl (main peak wavelength being near 175 nm), and F2 (main peak wavelength being near 153 nm). Here, the case in which the luminescent gas 15G is gas containing Xe is described.

As mentioned above, in the excimer lamp 11 of the present embodiment, the electrode 14a provided on the outer wall of the outer tube 13a has a mesh shape. Hence, there are openings in the electrode 14a, through which the ultraviolet light L1 is extracted toward the outside of the outer tube 13a. This ultraviolet light L1 is irradiated to the gas to be treated G1 flowing through the gas channel 5.

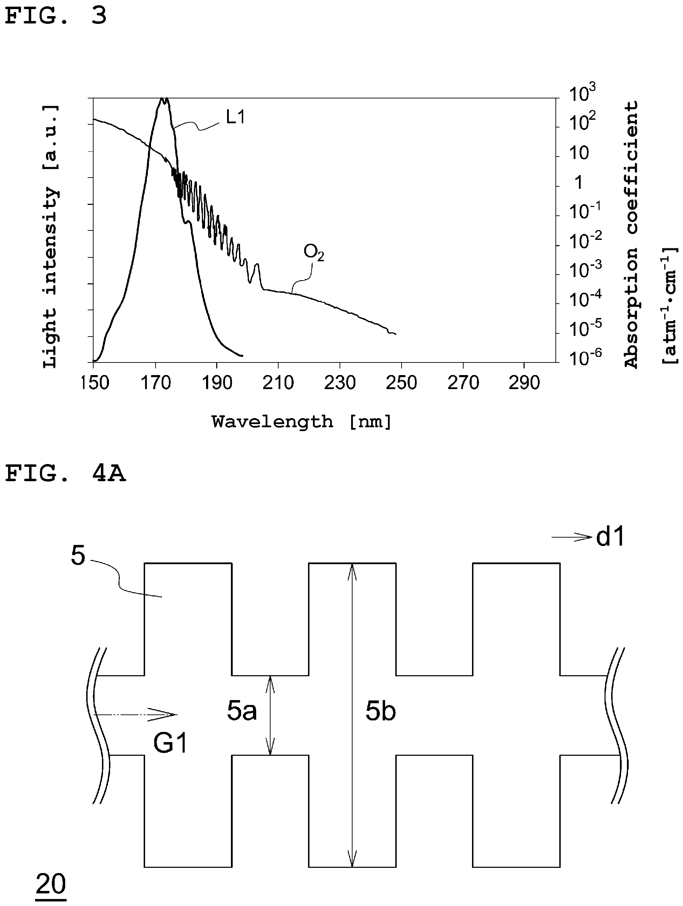

FIG. 3 is a graph indicating the spectrum of ultraviolet light L1 emitted from an excimer lamp 11 filled with luminescent gas 15G containing Xe and the absorption spectrum of oxygen (O2) in a superimposed manner. In FIG. 3, the horizontal axis represents the wavelength, the left vertical axis represents the relative value of the light intensity of the ultraviolet light L1, and the right vertical axis represents the absorption coefficient of oxygen (O2).

When gas containing Xe is used as the luminescent gas 15G of the excimer lamp 11, as shown in FIG. 3, the ultraviolet light L1 emitted from the excimer lamp 11 has a main peak wavelength of 172 nm, and the bandwidth in a range of approximately from 155 nm to 190 nm.

As described above, the gas to be treated G1 is air that is mixed with the target substance to be treated. Hence, when ultraviolet light L1 having a wavelength λ emitted from excimer lamp 11 is irradiated to the gas to be treated G1 and absorbed by oxygen (O₂), the reaction of the Formula (1) below proceeds. In Formula (1), O(¹D) represents an oxygen atom in the excited state and exhibits extremely high reactivity, while O(³P) represents an oxygen atom in the ground state. The reactions in Formulas (1) and (2) occur in response to the wavelength component of the ultraviolet light L1. Specifically, Formula (1) is the reaction that occurs when the shorter wavelength component of the ultraviolet light L1 is absorbed by oxygen (O₂). More precisely, Formula (1) is the reaction that occurs when ultraviolet light L1 having a wavelength λ of less than 175 nm is absorbed by oxygen (O₂). Formula (2) is the reaction that occurs when ultraviolet light L1 having a wavelength λ of 175 nm or more and less than 242 nm is absorbed.

O₂+ hv(λ) → O(¹D) + O(³P) ···· (1)

O₂+ hv(λ) → O(³P) + O(³P) ···· (2)

Part of the O atoms generated in Formulas (1) and (2) react with the oxygen (O₂) contained in the gas to be treated G1, thus which generates ozone (O₃) in accordance with Formula (3) below. In Formula (3), note that M indicates a third body. (the same applies hereinafter)

O + O₂ + M → O₃ + M ···· (3)

This reaction enables ozone to be introduced into the gas to be treated G1, thus the gas to be treated G1 changes to gas containing ozone. In other words, the excimer lamp 11 constitutes the ozone introduction unit in the gas treatment device 1 of the present embodiment.

From this viewpoint, the wall surface of the gas channel 5 through which the gas to be treated G1 flows is preferably made of ozone-resistant materials, for example stainless steel or glass. When the wall of the gas channel 5 is constituted by part of the housing 2, the housing 2 itself may be constituted by the aforementioned material.

The O(¹D) generated by the reaction in the above Formula (1) may react with water (H₂O) contained in the air to form hydroxyl radical in accordance with the Formula (4) below.

O(¹D) + H₂O → •OH + •OH ···· (4)

When the ultraviolet light L1 has a spectrum in the wavelength range of 185 nm or less, water vapor (H₂O) contained in the gas to be treated G1 absorbs the ultraviolet light L1 to produce hydroxyl radical (•OH) in accordance with Formula (5) below.

H₂O + hv(λ) → H + •OH .... (5)

Since extremely reactive, hydroxyl radical (•OH) may react with part of the target substance to be treated in the gas to be treated G1 and decompose it. However, hydroxyl radical (•OH) alone can decompose a very limited amount of the target substance to be treated contained in the gas to be treated G1. As an example, when methane (CH₄) is contained as the target substance to be treated, the hydroxyl radical (•OH) may cause the reaction in Formula (6) below to decompose methane.

CH₄ + •OH → CH₃ + H₂O .... (6)

In addition, part of the atomic oxygen, O(¹D) and O(³P), which are produced in the reactions of Formula (1) and (2), may react with the substance to be treated and decompose it, but still has a limited decomposition amount. As an example, when methane is contained as the target substance to be treated, methane may be decomposed by the reaction in Formula (7) below. Note that O(¹D) and O(³P) are collectively denoted as O in Formula (7).

CH₄ + O → CH₃ + •OH ... (7)

In accordance with the reactions shown in Formulas (6) and (7) above, part of the target substance to be treated undergoes the decomposition reaction with radicals, but this reaction alone makes it difficult to sufficiently decompose the target substance to be treated.

Hence, the gas treatment device 1 of the present embodiment includes a stirring area 20 that stirs the gas to be treated G1 flowing through the gas channel 5, and a heating area 30 that heats the gas to be treated G1 flowing through the gas channel 5. Both of the area are disposed downstream from a location of the area (ozone introduction area 10) in which ozone from the ozone introduction unit (excimer lamp 11) is introduced into the gas to be treated G1. Hereinafter, each of these area will be described.

### (Stirring area 20)

In the example of the gas treatment device 1 shown in FIG. 1, the stirring area 20 is constituted by arranging a plurality of wind shield plates 21 at separate locations along the direction of flow d1 in the gas channel 5. The gas to be treated G1 that collides with the wind shield plate 21 changes the direction of airflow due to the collision. Then, this causes part of the gas to be treated G1 to generate turbulence. As a result, the gas to be treated G1 is stirred with each other.

The wind shield plate 21, similar to the wall surface of the gas channel 5 through which the gas to be treated G1 flows, may be made of an ozone-resistant material, such as stainless steel, and glass.

Various methods can be employed to achieve the stirring area 20, in addition to providing the wind shield plates 21 in the gas channel 5. Examples may include, as shown in FIG. 4A, a method in which the shape of the gas channel 5 is set in a manner that the cross-sectional areas (5a, 5b) in the gas channel 5 are different at the position in the direction of flow d1. In this case, the direction of airflow of the gas to be treated G1 also changes at the locations in which the cross-sectional area of the gas channel changes, stirring the gas to be treated G1 with each other.

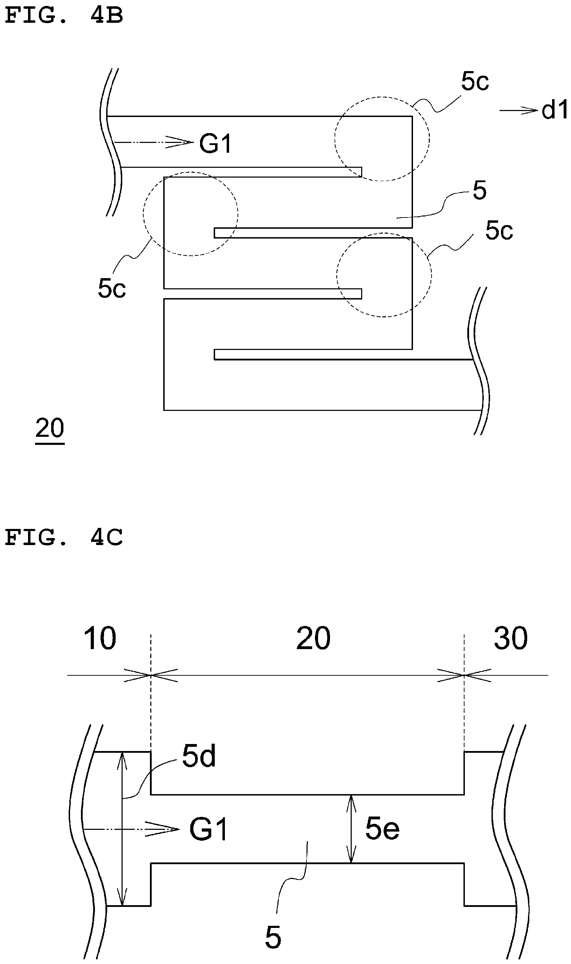

Another example includes, as shown in FIG. 4B, a method in which one or more bent sections 5c are provided in the stirring area 20 in the gas channel 5. In this case, the direction of airflow of the gas to be treated G1 also changes at the locations at which the bent sections 5c are formed, stirring the gas to be treated G1 with each other.

Yet another example includes, as shown in FIG. 4C, a method in which, while being the same in the stirring area 20, a cross-sectional area of the gas channel 5e is different in size compared to a cross-sectional area of the gas channel 5d in the ozone introduction area 10 that is located upstream from the stirring area 20. Since the cross-sectional area of the gas channel changes at a location at which the gas to be treated G1 flows from the ozone introduction area 10 into the stirring area 20, the direction of airflow of the gas to be treated G1 changes, thereby stirring the gas to be treated G1 with each other.

As described above, the stirring area 20 is provided for the purpose of stirring the gas to be treated G1 after the ozone has been introduced by the ozone introduction area 10. In other words, allowing the gas to be treated G1 flow through the stirring area 20 increases the probability of contact of the gas to be treated G1 with ozone. From the viewpoint of increasing this probability of contact, the stirring area 20 preferably has a longer length than the ozone introduction area 10 in the direction of flow d1 of the gas to be treated.

Incidentally, part of ozone (O₃) introduced by the ozone introduction area 10 may undergo the following decomposition reaction shown in Formula (8) below, when flowing through the stirring area 20 together with the gas to be treated G1.

O₃ → O(³P) + O₂ ···· (8)

When the above reaction occurs, highly reactive O(³P) reacts with the target substance to be treated contained in the gas to be treated G1 flowing through the stirring area 20, decomposing part of the target substance to be treated. In other words, the gas flowing through the stirring area 20 may have a mixture of the gas to be treated G1 that has been introduced from the gas inlet 3a, and the gas that has been treated G2 being in a state in which part of the target substance to be treated contained in the gas to be treated G1 has been decomposed. However, the rate of the ozone (O₃) decomposition reaction in Formula (8) that occurs in the stirring area 20 is sufficiently slow compared with the rate at which the gas to be treated G1 flows toward the heating area 30. Hence, when the gas to be treated G1 flows through the stirring area 20, the O(³P) that has been generated in the stirring area 20 can only decompose the substance to be treated to a very small extent. In the above viewpoint, the gas flowing from the stirring area 20 toward the heating area 30 is referred to as "gas to be treated G1".

### (Heating area 30)

As described above, the gas treatment device 1 shown in FIG. 1 is provided with a heating area 30 in which the gas to be treated G1 is heated at a position downstream from the stirring area 20. Here, part of the housing 2 constitutes a heating furnace 31, and the gas channel 5 is located in this heating furnace 31. The component constitutes the heating area 30.

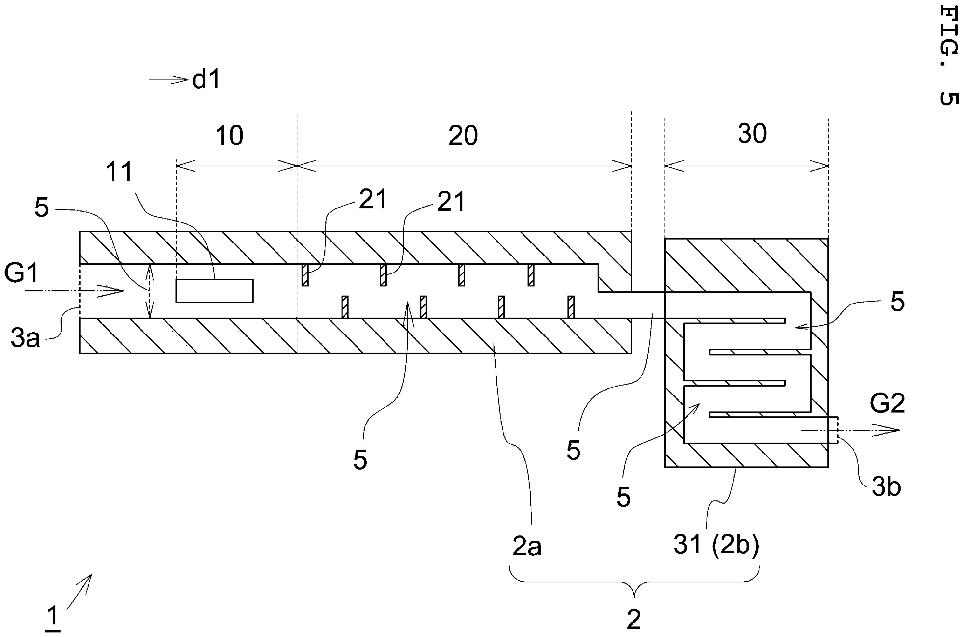

However, the heating area 30 does not necessarily need to be mounted in the same housing as the housing 2 into which the gas to be treated G1 flows. For example, as shown in FIG. 5, the configuration in which a housing 2a into which the gas to be treated G1 flows is communicated with the heating furnace 31 through the gas channel 5. In this case, the heating furnace 31 can be considered to constitute a kind of housing 2b; and the housing 2a, the housing 2b, and the gas channels 5 communicating these housings (2a, 2b) can be considered to form the single housing 2.

In other words, "housing 2" in the present specification is not limited to that exhibiting a single box shape; however, it is a concept entirely encompassing a structure that prevents the gas to be treated G1 and the gas that has been treated G2 from leaking out between the gas inlet 3a provided on one side and the gas outlet 3b provided on the other side. In other words, "housing 2" includes a structure in which a plurality of housings (2a, 2b, ...) are provided and each housing (2a, 2b, ...) is communicated with each other via the gas channels 5.

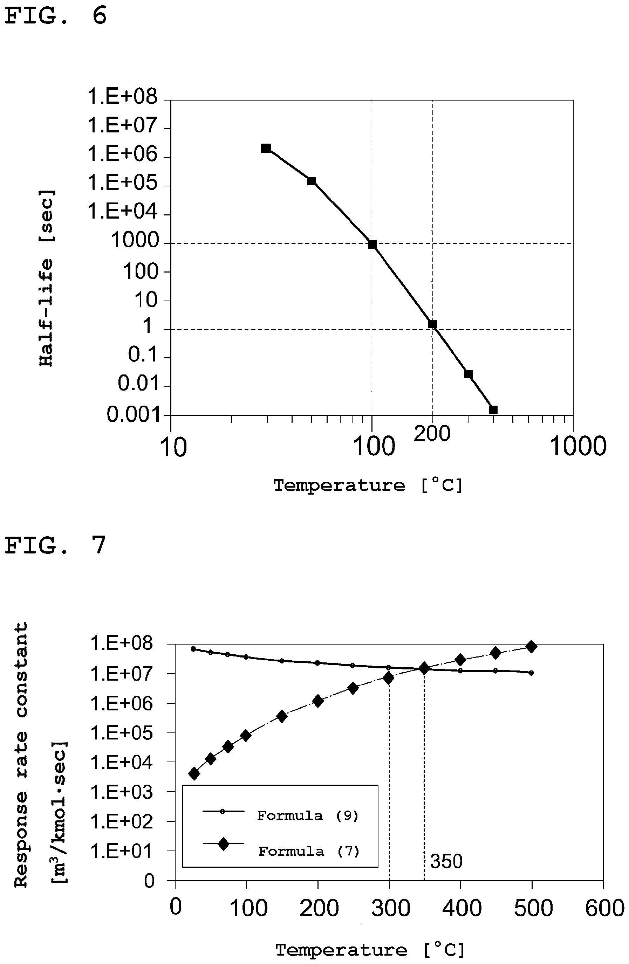

In the heating area 30, the gas to be treated G1 flowing through the gas channel 5 is heated to a temperature of 300°C or more. When being higher temperature, ozone (O₃) is known to make the reaction of the above Formula (8) proceed more due to thermal decomposition. FIG. 6 is a graph of the relationship between the thermal decomposition rate (half-life) of ozone and temperature. FIG. 6 shows that ozone has a half-life of approximately 1000 seconds at 100°C, and a half-life of less than 0.1 seconds at 300°C or higher. In other words, when ozone is heated at 300°C or higher, the reaction of Formula (7) proceeds on the half of the ozone present in 0.1 second or less, thus generating O(³P).

On the other hand, since the generated O(³P) is highly reactive, undergoes a reaction of changing to oxygen (O₂) in accordance with Formula (9) below if other substance M is present in the surroundings. Note that O(³P) is denoted as O, similar to Formula (7). Formula (7) is restated below for the sake of explanation.

O + O + M → O₂ + M ···· (9)

CH₄+ O → CH₃ + •OH ···· (7)

FIG. 7 is a graph illustrating the temperature dependence of the reaction rates of Formula (7) and Formula (9) above. As described above, increasing the temperature increases the reaction rate of Formula (8) and the amount of production of atomic oxygen (O). However, being highly reactive, atomic oxygen fails to contribute to decomposing target substance to be treated, and undergoes a reaction of changing to oxygen (O₂) in accordance with Formula (9). This reaction rate gradually decreases as the temperature increases.

In contrast, the reaction of Formula (7), i.e., the rate of the reaction in which the target substance to be treated is decomposed by atomic oxygen (O) increases as the temperature increases. When the target substance to be treated is methane (CH₄), atomic oxygen (O) reacts with the C-H bond to form the methyl radical (CH₃). In other words, the decomposition amount of methane in the gas to be treated G1 increases as the temperature of the gas to be treated G1 increases.

From the viewpoint of making the generated atomic oxygen (O) contribute more to decomposing the target substance to be treated, the gas to be treated G1 is preferably heated to 300°C or higher in the heating area 30. It is more preferably heated to 350°C or higher. Heating the gas to be treated G1 to 350°C or higher allows the reaction rate of Formula (7) to exceed that of Formula (9), thereby, further accelerating the decomposition of the target substance to be treated.

In general, a solid body has higher heating efficiency than gas. For this reason, the heating area 30 is set in a manner that the inner wall of the gas channel 5 present in the heating area 30 is heated. In other words, the gas channel 5 includes a heated wall. For example, in FIGS. 1 and 5, disposing the gas channel 5 in the heating furnace 31 supplies heat from the heating furnace 31 to the gas channel 5, making the temperature of the wall surface of gas channel 5 increase. Then, allowing the gas to be treated gas G1 to pass in the gas channel 5 with its wall surface being heated in this manner, increases the temperature of the gas to be treated G1.

The result of FIG. 7 indicates that the temperature of the gas to be treated G1 preferably increases as rapidly as possible. If the rate of temperature rise is slow, the reaction in which the generated atomic oxygen (O) changes to oxygen (O₂) in accordance with Formula (9) is likely to occur while the temperature of the gas to be treated G1 is increasing.

From the above viewpoint, the heating area 30 preferably has an aspect in which the gas to be treated G1 impinges multiple times at a certain velocity against the wall surface of the gas channel 5, which is heated to high temperature. The examples shown in FIGS. 1 and 5 illustrate a configuration in which the gas channel 5 is bent multiple times in the heating area 30. In this configuration, the gas to be treated G1 proceeds toward the gas outlet 3b while repeatedly and intermittently colliding the wall surface of the gas channel 5, thus the gas to be treated G1 can be rapidly heated to 300 °C or higher while flowing through the heating area 30.

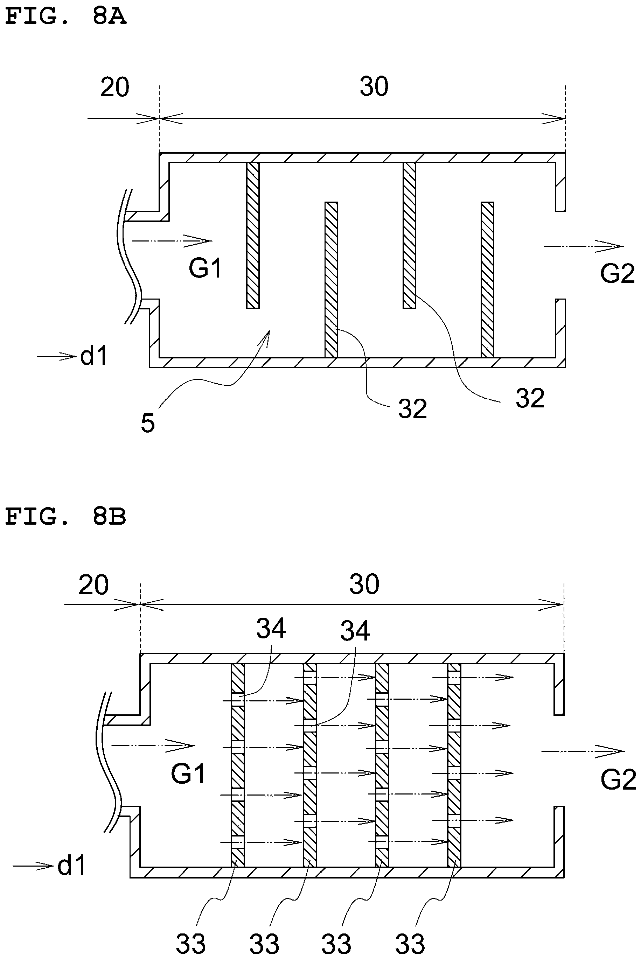

From the similar viewpoint, as shown in FIG. 8A, the heating area 30 may be provided with a plurality of heated plates 32 in a manner that the heated plates 32 are arranged to obstruct the direction of flow d1 while being separated from each other in the gas channel 5 in the direction of flow. The heated plates 32 are made of a material that can be heated to high temperature including metal materials such as stainless steel, aluminum, copper, silver, or ceramic, and are heated to 500°C, for example, by heat transfer from an external heat source. In this case, the gas to be treated G1 flowing through the heating area 30 proceeds toward the gas outlet 3b while repeatedly and intermittently colliding on the heated plates 32, thus the gas to be treated G1 can be heated rapidly in the same manner. Note that the heated plates 32 may be provided with a coating layer made of a material such as fluorine or ceramic, on its front surface to prevent degradation and rust due to ozone.

As yet another example, as shown in FIG. 8B, the heating area 30 can be configured such that the plurality of heated plates 33 each being provided with a plurality of flow holes 34 are arranged at positions spaced apart along the direction of flow d1. In this case, the position of the flow holes 34 is preferably set in a manner that the flow holes 34 of the adjacent heated plates 33 are not aligned each other along the direction of flow d1. In this case, the gas to be treated G1 flowing through the heating area 30 also proceeds toward the gas outlet 3b via the flow holes 34 while repeatedly and intermittently colliding on the heated plates 33, thus the gas to be treated G1 can be heated rapidly in the similar manner.

### (Another example of the configuration of an excimer lamp)

Note that the excimer lamp 11 provided as the ozone introduction unit in the present embodiment is not limited to the structure illustrated in FIGS. 2A and 2B.

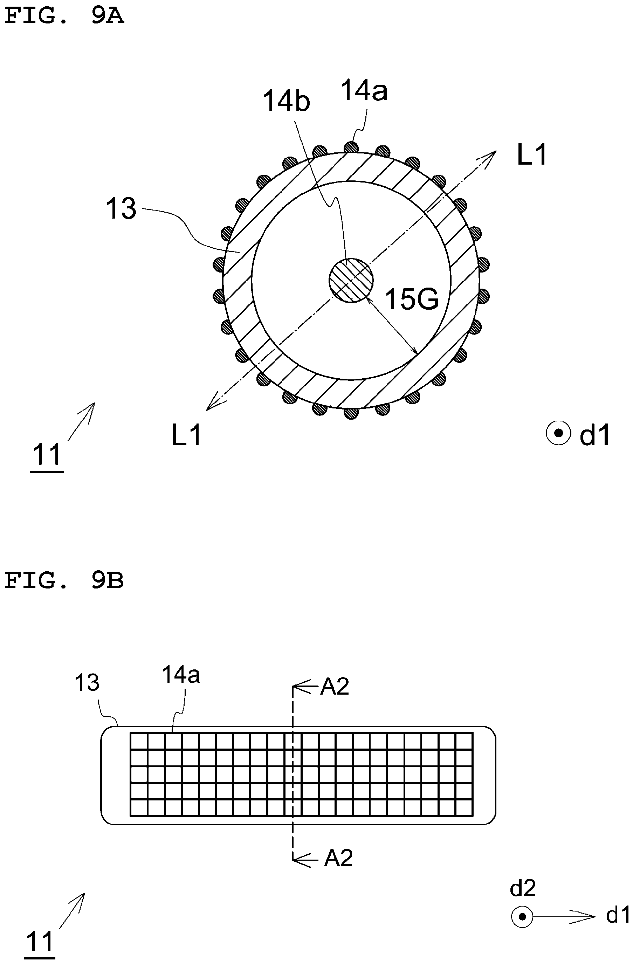

For example, as shown in FIG. 9A, the excimer lamp 11 may be provided with the single tube body 13. FIG. 9A is a schematic cross-sectional view of the excimer lamp 11 illustrated in accordance with FIG. 2B. The tube body 13 is sealed at its ends in the longitudinal direction, in other words, the direction of the tube axis d1 (not shown), and the luminescent gas 15G is sealed in the inner space of the tube body 13. The electrode 14a that has a mesh shape or a linear shape is provided on the outer wall surface of the tube body 13, and the rod-shaped electrode 14b is provided inside the tube body 13.

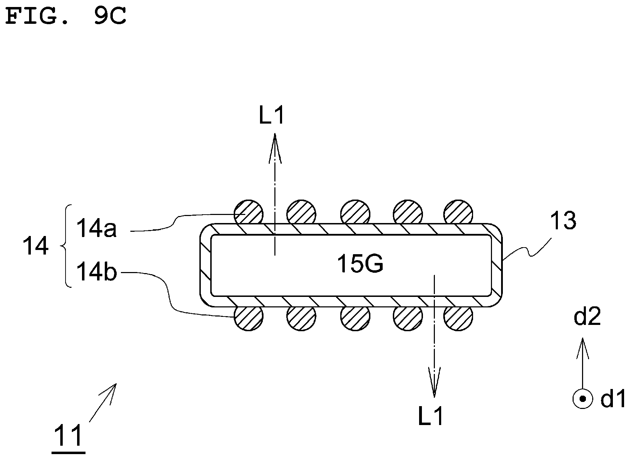

As another example, the configuration in which both of the electrodes (14a, 14b) are arranged on the outer wall surface of the tube body 13 of the excimer lamp 11 can also be employed. FIGS. 9B and 9C are drawings schematically illustrating the structure of the excimer lamp 11 in this another example of the configuration. FIG. 9B corresponds to a plan view, and FIG. 9C corresponds to a cross-sectional view taken along the line A2-A2 in FIG. 9B. The excimer lamp 11 shown in FIGS. 9B and 9C includes the single tube body 13, and both of the electrodes (14a, 14b) are arranged on the outer wall surface of the tube body 13 such that they are opposite each other via the tube body 13. The electrodes (14a, 14b) exhibits a mesh shape or a linear shape to allow the ultraviolet light L1 generated inside the tube body 13 to be extracted to the outside of the tube body 13.

### Second embodiment

A second embodiment of the gas treatment device according to the present embodiment will be described, focusing on the point that differs from that of the first embodiment. Elements that are common to the first embodiment will be assigned to the same symbol and their descriptions will be omitted as appropriate.

The gas treatment device 1 of the present embodiment differs from that of the first embodiment in the configuration of the ozone introduction unit.

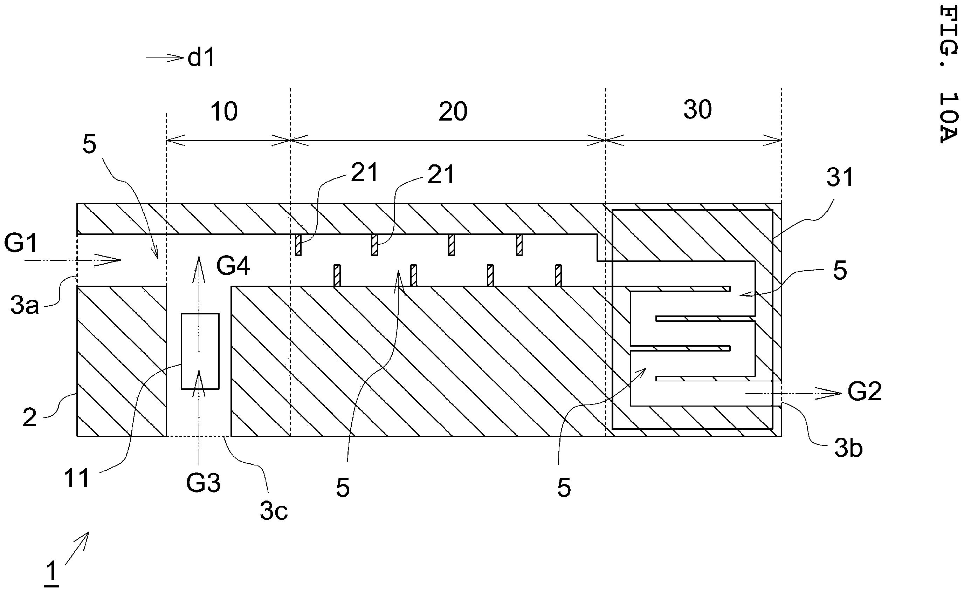

FIG. 10A is a cross-sectional view schematically illustrating the configuration of the second embodiment of the gas treatment device of the present invention in accordance with FIG. 1. In the present embodiment, the housing 2 is provided with a gas inlet 3c through which ozone source gas G3 is introduced, in addition to the gas inlet 3a through which the gas to be treated G1 is introduced. The ozone source gas G3 can be air that does not contain the target substance to be treated.

In this configuration, ozone gas G4 is generated from the ozone source gas G3 by excimer lamp 11 as an ozone introduction unit. This ozone gas G4 is introduced into the gas channel 5 through which the gas to be treated G1 flows. Since ozone is introduced into the gas to be treated G1 in the ozone introduction area 10, thus the gas to be treated G1 changes to gas containing ozone.

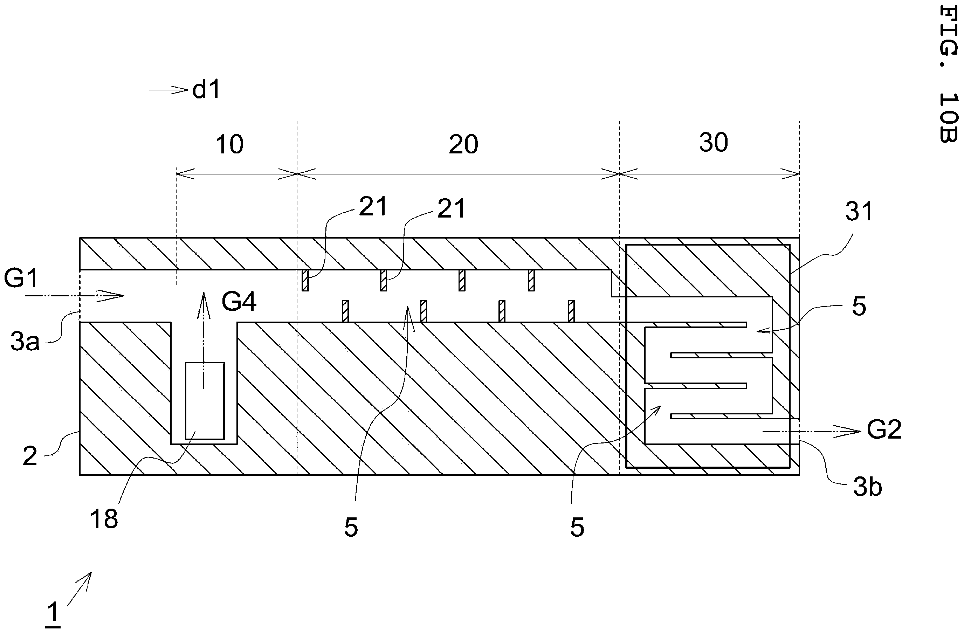

In the above viewpoint, the gas treatment device 1 according to the present invention can employ any ozone generation method provided that the gas treatment device 1 is configured such that ozone is introduced into the gas channel 5 through which the gas to be treated G1 flows in the ozone introduction area 10. Examples can include a configuration such that, as shown in FIG. 10B, an ozone cylinder 18 containing the ozone gas G4 is accommodated in the housing 2, and the ozone gas G4 supplied from this ozone cylinder 18 is introduced into the gas channel 5 through which the gas to be treated G1 flows.

### Other embodiment

In the embodiments described above, the excimer lamp 11 was referred to be described, as an example, as a device that generates ozone from air. However, instead of this excimer lamp 11, an atmospheric pressure plasma generator can be employed.

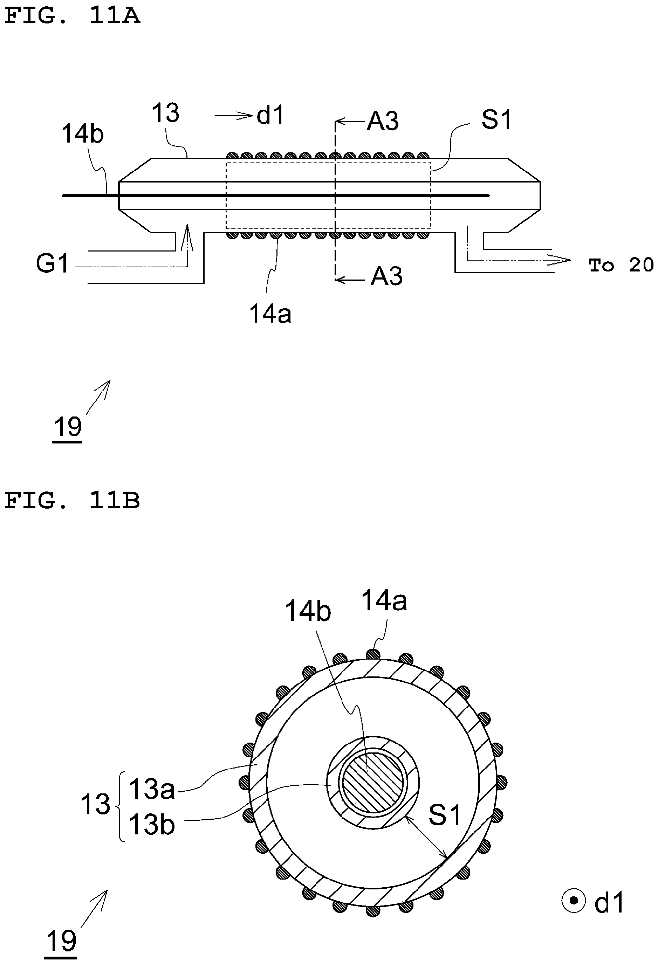

FIGS. 11A and 11B are drawings illustrating the schematic structure of the atmospheric pressure plasma generator. FIG. 11A corresponds to a side view of the atmospheric pressure plasma generator 19, and FIG. 11B corresponds to a cross-sectional view taken along the line A3-A3 in FIG. 11A.

The atmospheric pressure plasma generator 19 differs from the excimer lamp 11 in that the luminescent gas 15G is not sealed in the tube body 13. In FIG. 11A, the gas to be treated G1 is made to pass through the tube body 13. Other configurations are common to the excimer lamp 11 described above.

When the voltage is applied to both of the two electrodes (14a, 14b) from a power supply (not shown), a dielectric barrier discharge is generated in the tube body 13. This generates dielectric barrier discharge in the gas to be treated G1 flowing through the discharge space S1, allowing the gas to be treated G1 to transform into plasma.

Oxygen (O2) in the air contained in the gas to be treated G1 flows through the space of atmospheric pressure plasma, and undergoes the following reaction indicated in Formula (10) below. In Formula (10) below, AP means that energy is added by atmospheric pressure plasma.

O₂ + AP → O + O ···· (10)

Part of the oxygen atoms (atomic oxygen) O generated in Formula (10) reacts with oxygen (O2) contained in the gas to be treated G1, generating ozone (O3) in accordance with Formula (3) above. Formula (3) is restated below.

O + O₂ + M → O₃ + M ···· (3)

In other words, when the gas to be treated gas G1 flows through the atmospheric pressure plasma generator 19, ozone is introduced to the gas to be treated G1, and then the gas to be treated G1 changes to gas containing ozone.

The atmospheric pressure plasma generator 19 is not limited to the structures shown in FIGS. 11A and 11B. The atmospheric pressure plasma generator 19 can employ, for example, a structure similar to that of the excimer lamp 11 that is described above with reference to FIGS. 9A to 9C. In this case also, the tube body 13 may not be filled with the luminescent gas 15G, and the gas to be treated G1 may be made to flow through the tube body 13.

In addition, unlike the excimer lamp 11, the atmospheric pressure plasma generator 19 eliminates the need for extracting light to the outer wall surface of the tube body 13, hence the electrodes 14 that have been formed on the outer wall surface of the tube body 13 do not necessarily have a mesh shape or a linear shape.

Furthermore, instead of the excimer lamp 11 shown in FIG. 10A described in the second embodiment, the atmospheric pressure plasma generator 19 can also be employed.

However, when ozone is generated by the atmospheric pressure plasma generator 19, NOₓ components are unavoidably included due to the high energy from the plasma. Hence, in this method, NOₓ components are partly included in the gas that has been treated G2. When there are circumstances in which no NOₓ component is made to be included in the gas that has been treated G2, the excimer lamp 11 or the ozone cylinder 18 can preferably be used as the ozone introduction unit.

### Examples

The present invention will be described with reference to Examples; however the invention is not limited to these Examples.

### Description of experimental condition

Hereinafter, the experimental condition will be described.

### Experimental system

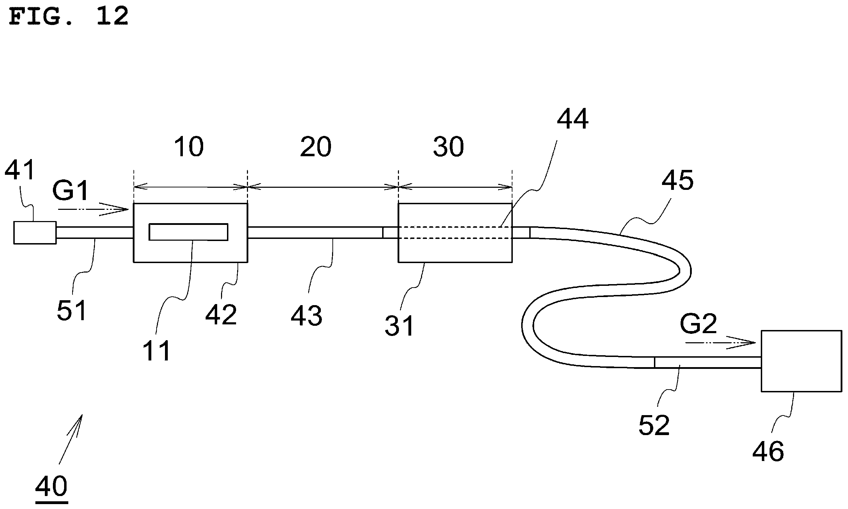

Experiments were conducted using an experimental system modeling the gas treatment device 1 of the first embodiment. FIG. 12 is a drawing schematically illustrating the structure of the experimental system.

A housing 42 accommodating the excimer lamp 11 therein shown in FIG. 9A was prepared as the ozone introduction area 10. The excimer lamp 11 had an outer diameter of 26 mm and a luminescent length of 200 mm. The housing 42 was a glass cylinder having an inner diameter (diameter) of 45 mm and a wall thickness of 3 mm, and its length was 350 mm. The excimer lamp 11 was disposed in the housing 42 with the tube axis of the tube body 13 being substantially aligned with the center axis of the housing 42. In the excimer lamp 11, the tube body 13 was filled with the luminescent gas 15G containing Xe, and the both of the electrodes (13a, 13b) were subject to a high frequency voltage having an input power of 40 W and an applied voltage of 8KVpp from a power supply (not shown), thereby emitting ultraviolet light L1 having a main peak wavelength of 172nm.

A glass tube 43 having a diameter smaller than the inner diameter of the housing 42 was prepared as the stirring area 20. The glass tube 43 had an inner diameter of 6 mm and a length of 1000 mm.

The heating furnace 31 constituted by an electric furnace was prepared as the heating area 30. The glass tube 43 as the stirring area 20 was connected with a heat-resistant glass tube 44 that was inserted in the heating furnace 31 at the front stage of the heating furnace 31. The heat-resistant glass tube 44 had an inner diameter of 6 mm and a length of 500 mm. The heating furnace 31 had a length of 300 mm along the direction of flow d1.

The heat-resistant glass tube 44 extended from the outlet of the heating furnace 31 was connected with a glass tube for cooling 45. The glass tube for cooling 45 had an inner diameter of 6 mm and a length of 1000 mm.

The glass tube for cooling 45 was connected with a sampling bag 46 via a flow tube 52. The flow tube 52 was constituted by a silicone tube, and had an inner diameter of 6 mm and a length of 3 m.

The gas to be treated G1 constituted by a mixed gas of air and methane (methane concentration of 100 ppm) was introduced from the gas inlet 3a, and allowed to pass through the ozone introduction area 10, the stirring area 20, and the heating area 30 in sequence. The resultant gas that had been treated G2 was introduced into the sampling bag 46 (manufactured by GL Sciences). Then, the gas that had been treated G2, which was contained in the sampling bag 46, underwent FTIR (Fourier Transform Infrared Spectroscopy) in order to analyze the components.

### Gas to be treated G1

The gas to be treated G1 having the components of conditions indicated in Table 1 below by FTIR analysis was employed. Note that Table 1 does not include nitrogen (N₂), oxygen (O₂), and argon (Ar) that are contained in the air in the gas to be treated G1. In addition, "NO_{y}" in Table 1 is a notation that collectively includes NO, NO₂, and N₂O, which are generally referred to as NOₓ, as well as N₂O₅, HNO₂, and HNO₃.

**Table 1**

| | CH₄ | CO | CO₂ | H₂O | NO_{y} | O₃ |
|---|---|---|---|---|---|---|
| Gas to be treated G1 [ppm] | 100.0 | 0.0 | 5.8 | 2906.4 | 0.0 | 0.0 |

### Examples 1 through 5

In Examples 1 through 5, the conditions were made such that the preset temperatures of the electric furnace were different, and the other conditions were the same.

### Comparative Example 1

In Comparative Example 1, the condition was such that no heating treatment was performed by turning off the power of the electric furnace. In other words, Comparative Example 1 corresponds to the case in which no heating treatment was performed on the gas to be treated G1 after ozone had been introduced.

### Comparative Example 2

In Comparative Example 2, the condition was such that the preset temperature of the electric furnace was the same as in Example 1, and no power is applied to the excimer lamp 11. In other words, Comparative Example 2 corresponds to the case in which the heat treatment was performed on the gas to be treated G1 without introducing ozone.

### Verification

Table 2 summarizes the experimental conditions under which each of Examples 1 through 5 and Comparative Examples 1 and 2 were conducted. In Table 2, note that the "temperature of the gas that has been treated T2" is the temperature of the gas that has been treated G2 measured at the outlet of the electric furnace. Under the experimental conditions shown in Table 2, the gas to be treated G1 having the components of the conditions shown in Table 1 was supplied from the gas cylinder 41 through the flow tube 51 toward the housing 42 at a flow rate of 10 L/min. The resultant gas that had been treated G2 was introduced into the sampling bag 46. The components of the gas that had been treated G2 were analyzed. The results are shown in Table 3.

**Table 2**

| | Flow rate of gas to be treated [L/min] | Ozone introduction | Heating | Stirring | Temperature of electric furnace T1 [°C] | Temperature of gas that has been treated T2 [°C] |
|---|---|---|---|---|---|---|
| Example 1 | 10 | Yes | Yes | Yes | 400 | 244 |
| Example 2 | 10 | Yes | Yes | Yes | 450 | 310 |
| Example 3 | 10 | Yes | Yes | Yes | 500 | 367 |
| Example 4 | 10 | Yes | Yes | Yes | 600 | 382 |
| Example 5 | 10 | Yes | Yes | Yes | 700 | 455 |
| Comparative Example 1 | 10 | Yes | No | Yes | - | 79 |
| Comparative Example 2 | 10 | No | Yes | No | 500 | 367 |

**Table 3**

| | | CH₄ | CO | CO₂ | H₂O | NO_{y} | O₃ |
|---|---|---|---|---|---|---|---|
| Gas to be treated G1 [ppm] | | 100.0 | 0.0 | 5.8 | 2906.4 | 0.0 | 0.0 |
| Gas that has been treated G2 [ppm] | Example 1 | 96.5 | 17.2 | 21.7 | 1007.2 | 0.0 | 2343.5 |
| | Example 2 | 96.0 | 14.2 | 15.8 | 859.6 | 0.0 | 2039.5 |
| | Example 3 | 94.2 | 14.0 | 13.9 | 912.3 | 0.0 | 1796.5 |
| | Example 4 | 86.8 | 18.9 | 12.6 | 1106.3 | 0.0 | 1463.8 |
| | Example 5 | 77.3 | 25.8 | 11.9 | 1050.0 | 0.0 | 985.6 |
| | Comparative Example 1 | 97.1 | 8.2 | 11.5 | 1643.5 | 0.0 | 2401.1 |
| | Comparative Example 2 | 99.5 | 0.0 | 6.8 | 899.9 | 0.0 | 2.2 |

### Analysis of result

Table 3 confirms that, in all cases, the concentration of methane in the gas that had been treated G2 was reduced compared to that in the gas to be treated G1. It was also confirmed that when the preset temperature of the electric furnace (heating furnace 31) was higher, the decomposition amount of methane became larger and the concentration of ozone mixed in the gas that had been treated G2 was lowered. FIG. 13 is a graph illustrating a relationship between the preset temperature of the electric furnace and the amount of methane and ozone remaining in the gas that has been treated G2, based on the results of Examples 1 through 5. In FIG. 13, the horizontal axis represents the preset temperature of the electric furnace, the left vertical axis represents the concentration of methane in the gas that has been treated G2, and the right vertical axis represents the concentration of ozone in the gas that has been treated G2.

In contrast, in Comparative Example 1, in which no heat treatment was performed, although being lower than that in the gas to be treated G1, the concentration of methane in the gas that had been treated G2 remains at higher concentrations with respect to those in each of Examples 1 through 5. In the case of Comparative Example 1, the ozone concentration is higher than those in Examples 1 to 5, indicating that ozone is not thermally decomposed compared to Examples 1 through 5. In other words, in Comparative Example 1, atomic oxygen (O) was not sufficiently generated, thus resulting in the insufficient decomposition amount of methane.

In the case of Comparative Example 2, in which the heat treatment was performed without introducing ozone, the methane contained in the gas that has been treated G2 was barely decomposed.

### Description of simulation conditions

Next, simulations were conducted to verify the difference in the decomposition efficiency of methane with and without the stirring area.

A cylinder (inner diameter: 50 mm) having the excimer lamp 11 therein was set as the ozone introduction area 10. The excimer lamp 11 having an outer diameter of 10 mm and a luminescent length of 300 mm was set. The heating area 30 having a length of 150 mm was set at the rear stage of the ozone introduction area 10.

When a mixed gas (methane concentration of 100 ppm) of air and methane as the gas to be treated G1 is made to flow through the introduction area 10 at a flow rate of 10 LPM, the methane concentration contained in the gas that has been treated G2 was calculated. Specifically, the concentration of methane in the gas that has been treated G2 was calculated in the case in which the heating area 30 is provided immediately after the ozone introduction area 10 (condition #1) and in the case in which the stirring area 20 having a cylinder is provided between the ozone introduction area 10 and the heating area 30 (Condition #2). Comparing these results can confirm the degree to which the presence of the stirring area 20 affects the capability of decomposition and production of methane.

Two patterns were set for the heating area 30 such that the case in which the ambient temperature is 450°C and the case in which the ambient temperature is 700°C. The heating area 30 was simulated as an area in which the cylinder through which the mixed gas (gas to be treated G1) flows is disposed in the electric furnace, the area being similar to the ozone introduction area 10.

Condition #1 was set as follows. The ozone introduction area 10 was set to an area with a length of 300 mm in the longitudinal direction (the direction of flow of the gas to be treated G1) in the cylinder having an inner diameter of 50 mm. The heating area 30 was set to an area with a length of 150 mm in the longitudinal direction in the cylinder having an inner diameter of 50 mm, the area being disposed immediately after the ozone introduction area 10.

Condition #2 was set as follows. The ozone introduction area 10 was set to an area with a length of 300 mm in the longitudinal direction in the cylinder having an inner diameter of 20 mm. In the cylinder having an inner diameter of 20 mm, the stirring area 20 was set to an area with a length of 300 mm in the longitudinal direction immediately after the ozone introduction area 10, and furthermore the heating area 30 was set to be an area with a length of 150 mm disposed immediately after the stirring area 20.

As described above, the length of the heating area 30 in the longitudinal direction is set to the same value (150 mm) in order to make the heating conditions common in Condition #1 and Condition #2.

The results of verification are shown in Table 4.

| | Decomposition amount of methane [ppm] | |
|---|---|---|
| | 450°C | 700°C |
| #1 (without stirring area) | 5.1 | 11.8 |
| #2 (with stirring area) | 8.1 | 17.5 |
| Difference in decomposition rate | 3.0% | 5.7% |

Note that the "Difference in decomposition rate" in Table 4 refers to the value that is calculated by dividing the value of difference in decomposition amount of methane with and without the stirring area by the initial methane concentration (100 ppm).

Table 4 confirms that, when compared under the same heating temperature, the decomposition amount of methane on the treatment under Condition #2 (with the stirring area) is higher than that on the treatment under Condition #1 (without the stirring area).

### The reasons for this are inferred as follows.

In Condition #1, since no stirring area 20 is provided between the ozone introduction area 10 and the heating area 30, the mixed gas is introduced into the heating area 30 with insufficient stirring of the ozone and methane. This causes atomic oxygen that has been produced by the thermal decomposition of ozone to disappear at a higher rate before colliding with methane. In contrast, in condition #2, since ozone is introduced to the gas to be treated G1 and then the mixed gas flows through the stirring area 20, the stirring of ozone and methane is promoted. The mixed gas in the state that ozone and methane have been stirred through the stirring area 20 is introduced into the heating area 30, thereby increasing the probability of collision between the atomic oxygen that has been obtained by the thermal decomposition of ozone and the methane.

Table 4 also confirms that increasing the heating temperature widens the difference of decomposition capability of methane between Condition #2 (with the stirring area) and Condition #1 (without the stirring area).

The reasons for this are inferred as follows. Since the thermal decomposition of ozone is accelerated as the mixed gas becomes at high temperatures, the amount of atomic oxygen produced by the thermal decomposition of ozone in the case of a heating temperature of 700°C is higher than that in the case of a heating temperature of 450°C. However, as described above, the probability that atomic oxygen disappears without colliding with methane is higher in the case of Condition #1, in which no stirring area 20 is provided, compared to Condition #2, in which the stirring area 20 is provided. As a result, setting the higher heating temperature increases the amount of atomic oxygen that disappears without colliding with ozone, which is caused by the absence of the stirring area 20.

In other words, with the heating temperature in the heating area 30 being set higher, providing the stirring area 20 can significantly enhance the performance of decomposition of methane.

As described above, introducing ozone and undergoing heating treatment after stirring enables the target substance to be treated that has been contained in the gas to be treated G1 to be in contact with atomic oxygen (O) at a high probability, increasing the decomposition rate of the target substance to be treated. In particular, the above results confirm that methane that has been mixed with air at an extremely low concentration of 100 ppm can also be decomposed at a high rate.

In addition, the effect of providing the stirring area 20 is particularly confirmed to be noticeable in the equipment in which the thermal decomposition of ozone can be effectively performed.

### Reference Signs List

- 1: Gas treatment device
- 2: Housing
- 2a: Housing
- 2b: Housing
- 3a: Gas inlet
- 3b: Gas outlet
- 3c: Gas inlet
- 5: Gas channel
- 5c: Bent section
- 5d: Cross-sectional area of gas channel
- 5e: Cross-sectional area of gas channel
- 5f: Cross-sectional area of gas channel
- 10: Ozone introduction area
- 11: Excimer lamp
- 13: Tube body
- 13a: Outer tube
- 13b: Inner tube
- 14: Electrode
- 14a: Electrode
- 14b: Electrode
- 15G: Luminescent gas
- 18: Ozone cylinder
- 19: Atmospheric pressure plasma generator
- 20: Stirring area
- 21: Wind shield plate
- 30: Heating area
- 31: Heating furnace
- 32: Heated plate
- 33: Heated plate
- 34: Flow hole
- 40W: Power
- 41: Gas cylinder
- 42: Housing
- 43: Glass tube
- 44: Heat-resistant glass tube
- 45: Glass tube for cooling
- 46: Sampling bag
- 51: Flow tube
- 52: Flow tube
- 100: Methane removal system
- 101: Emission source of gas to be treated
- 102: Tube for gas to be treated
- 102a: Channel for gas to be treated
- 104: Catalyst for removing methane by oxidation
- 104a: Catalyst container
- 105: Plasma generation means
- 106: Control means
- 107: Power supply source
- 108a: External electrode
- 108b: Internal electrode
- G1: Gas to be treated
- G2: Gas that has been treated
- G3: Ozone source gas
- G4: Ozone gas
- L1: Ultraviolet light
- D1: Direction of flow

## Claims

1. A gas treatment method comprising:
a process (a) of allowing gas to be treated in which a target substance to be treated is mixed with air to pass through inside a housing, the target substance to be treated exhibiting volatility at room temperature and belonging to at least one substance selected from a group consisting of carbon compounds, nitrogen compounds, and sulfur compounds;
a process (b) of introducing ozone into a space through which the gas to be treated flows inside the housing at 200°C or lower;
a process (c) of stirring the gas to be treated after the process (b); and
a process (d) of heating the gas to be treated to 300°C or higher after executing the process (c).

2. The gas treatment method according to claim 1, wherein the process (c) is a process of allowing the gas to be treated to pass through a stirring area in which a cross-sectional area of a gas channel is varied, or a stirring area in which a wind shield plate is provided in the middle of the gas channel.

3. The gas treatment method according to claim 2, wherein the stirring area has a length longer than a length of an area through which the gas to be treated flows when the process (b) is executed, in a direction of flow of the gas to be treated.

4. The gas treatment method according to any one of claims 1 to 3, wherein the target substance belongs to at least one substance selected from a group consisting of methane, acetylene, ethylene, ethane, propane, methylamine, ammonia, hydrogen sulfide, and methyl mercaptan.

5. A gas treatment device is a gas treatment device that treats gas to be treated in which a target substance to be treated is mixed with air, the target substance to be treated exhibiting volatility at room temperature and belonging to at least one substance selected from a group consisting of carbon compounds, nitrogen compounds, and sulfur compounds, the gas treatment device comprising:
a housing;
a gas inlet through which the gas to be treated is introduced into the housing;
an ozone introduction unit that introduces ozone into a gas channel through which the gas to be treated flows inside the housing;
a stirring area that is disposed downstream from a location of the ozone introduction unit, and that stirs the gas to be treated flowing through the gas channel;
a heating area that is disposed downstream from a location of the stirring area, and that heats the gas to be treated flowing through the gas channel; and
a gas outlet through which gas that has been treated having passed through the heating area is exhausted from the housing.

6. The gas treatment device according to claim 5, wherein the gas channel in the stirring area in the direction of flow of the gas to be treated has a length longer than a length from a location at which ozone is introduced by the ozone introduction unit to the stirring area in the direction of flow of the gas to be treated.

7. The gas treatment device according to claim 6, wherein the gas channel is configured to include a heated wall whose surface is heated to 300°C or higher in the heating area.

8. The gas treatment device according to claim 7, wherein the gas channel is bent in the heating area.

9. The gas treatment device according to any one of claims 5 to 8, wherein the gas channel is configured in a manner that a cross-sectional area of the gas channel changes in the stirring area.

10. The gas treatment device according to any one of claims 5 to 8, wherein the gas channel includes a wind shield plate with which the gas to be treated flowing through the gas channel collides in the stirring area.

11. The gas treatment device according to any one of claims 5 to 8, wherein the ozone introduction unit includes a light source that is disposed in the gas channel, and that emits ultraviolet light having a main peak wavelength of less than 200 nm, and ozone is generated from part of the gas to be treated by irradiating the gas to be treated with the ultraviolet light from the light source.

12. The gas treatment device according to any one of claims 5 to 8, wherein the ozone introduction unit includes an atmospheric pressure plasma generator that is disposed in the gas channel, and ozone is generated from part of the gas to be treated by allowing the gas to be treated to pass through a space of atmospheric pressure plasma generated by the atmospheric pressure plasma generator.

13. The gas treatment device according to any one of claims 5 to 8, wherein the ozone introduction unit includes an ozone generator that is disposed in a channel different from the gas channel, and ozone gas generated from the ozone generator is supplied into the gas channel.
